(19) Europäisches Patentamt
European Patent Office
Office européen des brevets

(11) EP 2 171 077 B1

(12) **EUROPEAN PATENT SPECIFICATION**

(45) Date of publication and mention of the grant of the patent:
**28.11.2012 Bulletin 2012/48**

(21) Application number: **08775048.5**

(22) Date of filing: **11.07.2008**

(51) Int Cl.:
*C12P 7/22* (2006.01) *C12P 7/24* (2006.01)
*C12P 13/02* (2006.01) *C12P 41/00* (2006.01)

(86) International application number:
**PCT/EP2008/059143**

(87) International publication number:
**WO 2009/007460 (15.01.2009 Gazette 2009/03)**

(54) **Enantioselective reduction**

Enantioselektive Reduktion

Réduction énantiosélective

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.07.2007 EP 07013560**

(43) Date of publication of application:
**07.04.2010 Bulletin 2010/14**

(73) Proprietor: **DSM IP Assets B.V.**
**6411 TE Heerlen (NL)**

(72) Inventors:
- **WILDEMAN DE, Stefaan Marie André**
  **B-3630 Maasmechelen (BE)**
- **STRAATMAN, Henricus Martinus Maria Gerardus**
  **NL-5961 LG Horst (NL)**
- **VERZIJL, Gerardus Karel Maria**
  **NL-5855 AR Well (NL)**
- **VERMOTE, Linda**
  **NL-6137HB Sittard (NL)**
- **VRIES DE, Andreas Hendrikus Maria**
  **NL-6212 CR Maastricht (NL)**

(74) Representative: **Hoogendam, Gerrie Christine**
**DSM Intellectual Property**
**P.O. Box 4**
**6100 AA Echt (NL)**

(56) References cited:
**DE-A1-102005 063 191**

- **HALL ET AL: "Asymmetric whole-cell bioreduction of an alpha,beta-unsaturated aldehyde (citral): competing prim-alcohol dehydrogenase and C-C lyase activities" TETRAHEDRON: ASYMMETRY, PERGAMON, OXFORD, GB, vol. 17, no. 21, 7 December 2006 (2006-12-07), pages 3058-3062, XP005796924 ISSN: 0957-4166**
- **HALL MÉLANIE ET AL: "Asymmetric bioreduction of activated alkenes using cloned 12-oxophytodienoate reductase isoenzymes OPR-1 and OPR-3 from Lycopersicon esculentum (tomato): a striking change of stereoselectivity." ANGEWANDTE CHEMIE (INTERNATIONAL ED. IN ENGLISH) 2007, vol. 46, no. 21, May 2007 (2007-05), pages 3934-3937, XP002465483 ISSN: 1433-7851 cited in the application**
- **MUELLER ANDRE ET AL: "Asymmetric alkene reduction by yeast old yellow enzymes and by a novel Zymomonas mobilis reductase" BIOTECHNOLOGY AND BIOENGINEERING, [Online] vol. 98, no. 1, September 2007 (2007-09), pages 22-29, XP002465486 ISSN: 0006-3592 [retrieved on 2007-03-09]**
- **MUELLER A ET AL: "Enzymatic reduction of the alpha,beta-unsaturated carbon bond in citral" JOURNAL OF MOLECULAR CATALYSIS. B, ENZYMATIC, ELSEVIER, AMSTERDAM,, NL, vol. 38, no. 3-6, March 2006 (2006-03), pages 126-130, XP002419723 ISSN: 1381-1177**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YANG JUNG WOON ET AL: "Metal-free, organocatalytic asymmetric transfer hydrogenation of alpha,beta-unsaturated aldehydes." ANGEWANDTE CHEMIE (INTERNATIONAL ED. IN ENGLISH) 17 DEC 2004, vol. 44, no. 1, 17 December 2004 (2004-12-17), pages 108-110, XP002465487 ISSN: 1433-7851
- KOMDUUR JANET A ET AL: "Old yellow enzyme confers resistance of Hansenula polymorpha towards allyl alcohol" CURRENT GENETICS, vol. 41, no. 6, September 2002 (2002-09), pages 401-406, XP002465490 ISSN: 0172-8083
- MANO JUN'ICHI ET AL: "The NADPH:quinone oxidoreductase P1-zeta-crystallin in Arabidopsis catalyzes the alpha,beta-hydrogenation of 2-alkenals: detoxication of the lipid peroxide-derived reactive aldehydes." PLANT & CELL PHYSIOLOGY DEC 2002, vol. 43, no. 12, December 2002 (2002-12), pages 1445-1455, XP002465912 ISSN: 0032-0781 cited in the application
- DICK RYAN A ET AL: "Antioxidative function and substrate specificity of NAD(P)H-dependent alkenal/one oxidoreductase. A new role for leukotriene B4 12-hydroxydehydrogenase/15-oxoprostaglandi n 13-reductase" JOURNAL OF BIOLOGICAL CHEMISTRY, vol. 276, no. 44, 2 November 2001 (2001-11-02), pages 40803-40810, XP002465491 ISSN: 0021-9258
- WANNER PETER ET AL: "Purification and characterization of two enone reductases from Saccharomyces cerevisiae" EUROPEAN JOURNAL OF BIOCHEMISTRY, BERLIN, DE, vol. 255, no. 1, July 1998 (1998-07), pages 271-278, XP002202649 ISSN: 0014-2956
- RUEGER H ET AL: "A convergent synthesis approach towards CGP60536B, a non-peptide orally potent renin inhibitor, via an enantiomerically pure ketolactone intermediate" TETRAHEDRON LETTERS, ELSEVIER, AMSTERDAM, NL, vol. 41, no. 51, 16 December 2000 (2000-12-16), pages 10085-10089, XP004225222 ISSN: 0040-4039
- VAZ A D N ET AL: "OLD YELLOW ENZYME: AROMATIZATION OF CYCLIC ENONES AND THE MECHANISM OF A NOVEL DISMUTATION REACTION" BIOCHEMISTRY, AMERICAN CHEMICAL SOCIETY. EASTON, PA, US, vol. 34, no. 13, 1995, pages 4246-4256, XP001164161 ISSN: 0006-2960
- SHIH T W ET AL: "Nonenzymatic isomerization of 9-cis-retinoic acid catalyzed by sulfhydryl compounds." DRUG METABOLISM AND DISPOSITION: THE BIOLOGICAL FATE OF CHEMICALS JAN 1997, vol. 25, no. 1, January 1997 (1997-01), pages 27-32, XP002465488 ISSN: 0090-9556
- DATABASE WPI Week 199502 Thomson Scientific, London, GB; AN 1995-011795 XP002465499 & JP 06 298726 A (KURARAY CO LTD) 25 October 1994 (1994-10-25)
- WOLKEN W A ET AL: "Amino acid-catalyzed conversion of citral: cis-trans isomerization and its conversion into 6-methyl-5-hepten-2-one and acetaldehyde." JOURNAL OF AGRICULTURAL AND FOOD CHEMISTRY NOV 2000, vol. 48, no. 11, November 2000 (2000-11), pages 5401-5405, XP002465497 ISSN: 0021-8561
- DATABASE WPI Week 200417 Thomson Scientific, London, GB; AN 2004-180135 XP002465500 & WO 03/082895 A (PHARMACIA & UPJOHN CO) 9 October 2003 (2003-10-09)
- ZAGOZDA ET AL: "Enantioselective reduction of alpha,beta-unsaturated ketones by Geotrichum candidum, Mortierella isabellina and Rhodotorula rubra yeast" TETRAHEDRON: ASYMMETRY, PERGAMON, OXFORD, GB, vol. 17, no. 13, 14 August 2006 (2006-08-14), pages 1958-1962, XP005602166 ISSN: 0957-4166
- STUERMER ET AL: "Asymmetric bioreduction of activated C=C bonds using enoate reductases from the old yellow enzyme family" CURRENT OPINION IN CHEMICAL BIOLOGY, CURRENT BIOLOGY LTD, LONDON, GB, vol. 11, no. 2, 5 April 2007 (2007-04-05), pages 203-213, XP022022266 ISSN: 1367-5931

## Description

The invention relates to a process for the enantioselective reduction of an α,β-unsaturated compound.

Examples of enantioselective reduction have been described in scientific literature. For example, Fardelone et al., in J. Mol. Catal. B: Enzymatic 29 (2004) 41-45 describe the enantioselective reduction of α-substituted (E)-cinnamaldehyde mediated by baker's yeast, yielding enantiopure α-substituted-3-phenyl-1-propanol.

Ferraboschi et al., in Tetrahedron:Asymmetry 10(1999) 2639 describe the preparation of 2-methyl-1-alkanols by yeast mediated enantioselective biocatalytic reduction of unsaturated compounds containing an methylene group.

Mano et al., in Plant & Cell Physiology, 43(12):1445-1455 (2002), describe the use of an NADPH:Quinone oxidoreductase to convert an alkenal in a aldehyde, using NADPH as a cofactor.

Hall et al., in Angewandte Chemie 2007, 46, 3934-3937, describe the asymmetric reduction of C=C bonds of several unsaturated compounds by an enoate reductase.

Müller et al., Biotechnology and Bioengineering, vol. 98, no. 1 (2007): 22-29 describe the asymmetric alkene reduction by yeast old yellow enzymes and by a *Zymomonas mobilis* reductase. Enantiomers of citronellal, 3-methylcyclohexanone, 2-methylpentanal and 2-methyl-3-phenylpropanal are shown to be formed from the corresponding α,β-unsaturated substrates by enzymatic reduction.

In the processes described in the literature referred to above, only the conversion of E-isomers is shown.

The inventors realised that it would be desirable to provide a process wherein both E and Z isomers are converted to a desired enantiomer, whereby a wider substrate spectrum is obtained.

Surprisingly, the inventors found it to be possible to convert both the E and the Z isomer of an α,β-unsaturated compound into a chiral compound by using an ene reductase under specific reaction conditions.

Thus, the invention relates to a process for the preparation of a chiral compound, of formula 3 comprising enantioselectively converting a carbon-carbon double bond of an α,β-unsaturated compound of formula 2 in a mixture comprising both an E isomer and a Z isomer of the α,β-unsaturated compound, wherein both E isomer and Z isomer are converted in the presence of an ene reductase under isomerising conditions according to claim 1.

The compound obtained after the enantioselective reduction of the α,β-unsaturated compound contains at least one stereogenic centre. In particular the compound may comprise one, two or more stereogenic centres. The compound obtained may be a racemic mixture, a diastereomeric mixture, or be scalemic *i.e.* enantio-enriched (*i.e.* one of the enantiomers is formed in excess of the other but not to the exclusion of the other) or enantiopure (*i.e.* essentially only one of the enantiomers is formed). Thus, the compound may be the 2S, 3S diastereomer, 2S, 3R diastereomer 2R, 3S diastereomer 2R, 3R diastereomer or a mixture thereof.

The term "enantioselective" is used to indicate that one of the enantiomers is formed in excess of the other, or even to the exclusion of the other. (The latter often being referred to as enantiospecific.) Thus, in an enantioselective process of the invention the Z isomer and the E isomer are both preferentially converted into the same enantiomer. Usually, the enantioselectivity is such that one of the enantiomers is formed with enantiomeric excess (ee) of at least 40 %, in particular of at least 50 %, more in particular of at least 60 %, even more in particular of at least 70 %. Preferably, the enantioselectivity is such that one of the enantiomers is formed with an ee of at least 80 %, or of at least 85 %. The ee for one of the enantiomers may in particular be about 90 % or more, or about 95 % or more. Enantiomeric excess of an enantiomer (enantiomer 1) is a measure for how much of one of the enantiomers is present compared to the corresponding enantiomer (enantiomer 2) and is defined as follows.

$$ee = [C_{enantiomer\ 1} - C_{enantiomer\ 2}]/[C_{enantiomer\ 1} + C_{enantiomer\ 2}] \times 100\ \%$$

Herein "$C_{enantiomer\ 1}$" is the molar concentration of the enantiomer of which ee is determined and "$C_{enantiomer\ 2}$ "is the molar concentration of the corresponding enantiomer.

As the invention allows conversion of both an E isomer and a Z isomer of an unsaturated compound to a desired enantiomer, the conversion of an isomeric mixture of an E isomer and a Z isomer towards a desired enantiomer is higher than in the known processes wherein only one of the isomers would be converted if such known process would be applied to such a mixture of the E isomer and the Z isomer. It has been found possible to choose conditions such that both E isomer and Z isomer are converted with a good yield for a desired enantiomer. At least for some compounds it has been found possible to substantially completely convert both the E isomer and the Z isomer. As used herein, a conversion is in particular considered substantially complete for a specific isomer if at least 90 % of that isomer, in particular

at least 95 % of that isomer is converted to the desired chiral compound.

Thus, if desired, a product may be obtained which may be used, e.g. in a further reaction for preparing a pharmaceutical compound or an intermediate for a pharmaceutical compound, without further purification or after a simpler purification than needed for a product obtained by a known process to obtain a product of similar purity, Moreover,

an extra purification step may lead to product loss, and thus reduced overall yield of the product.

The mixture comprising the E isomer and the Z isomer usually has a molar ratio of Z isomer to E isomer of 1:99 to 99:1. In particular, the molar ratio of Z isomer to E isomer may be in the range of 5:95 to 95:5, preferably in the range of 10:90 to 90:10, in particular of 20:80 to 80:20, more in particular of 25:75 to 75:25. A process of the invention offers in particular an advantage in that the Z isomer also can be converted enantioselectively, which makes a method of the invention also particularly interesting in case the mixture which is to be converted comprises a high amount of Z isomer, such as mixture with a molar ratio of Z isomer to E isomer at least 50:50.

The $\alpha,\beta$-unsaturated compound represented by Formula 2 and its corresponding isomer may be converted

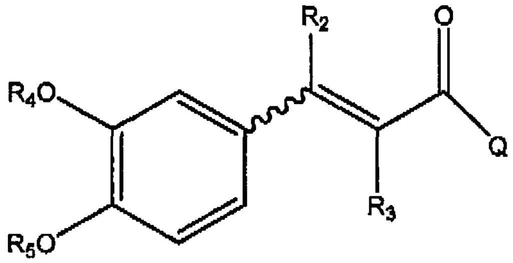

(2)

wherein $R_2$ is H; $R_3$ is an alkyl group comprising between 1 and 12 C-atoms;
$R_4$ is selected from the group of hydrogen, $C_1$-$C_6$ alkyls, $C_2$-$C_6$ alkoxyalkyls and oxygen protective groups; $R_5$ is selected from the group of hydrogen, $C_1$-$C_6$ alkyl or an oxygen protective group; Q is H,
to provide a compound with formula (3)

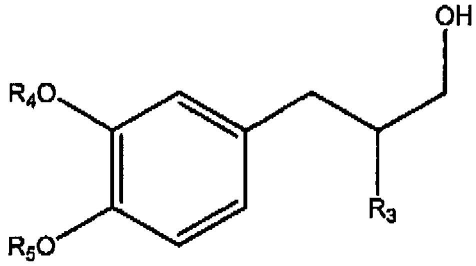

(3)

wherein $R_3$, $R_4$ and $R_5$ are as described above for formula (2).

Within the context of the present invention the term "oxygen protective group" is used to describe any group suitable to protect an oxygen of an alcohol against an undesired reaction. In particular, the oxygen protective group may be selected from the group of tosylate, mesylate, benzoylate, benzoate, trihydrocarbon-silyl, e.g. trimethyl-silyl, and organic acid residues, such as acetate.

It is in particular surprising that the specific reagent facilitating isomerisation can be used in the presence of the 2) without unacceptably detrimentally affecting the activity of the ene reductase. Thus, when using said reagent, there is no need to first transform the isomer that is not or to a lesser extent converted by the ene reductase into the active (or more active) isomer, and thereafter in an isolated process step, to reduce the isomer. Thus, the process can advantageously take place in a one-pot process, while still allowing a maximum theoretical yield of the desired chiral compound of 100%, based on the amount of starting isomers. Apart from the fact that such process can be employed making use of a simpler reactor system, the isomerisation may take place more efficiently, e.g. quicker and/or more complete.

The process according to the invention is carried out under isomerising conditions. Isomerising conditions are defined as conditions under which the E and Z isomers of the starting material are converted into each other. Isomerising conditions are provided by carrying out the reduction in the presence of a compound capable of participating in a Michael addition and retro-Michael addition with the unsaturated compount, wherein the compound is selected from the group of alkane thiols, *e.g.*, ethane thiol, propane thiol or butane thiol and aryl thiols, *e.g.* thiophenol.

In particular, a thio-alcohol may be used as a compound capable of participating in a Michael addition and/or retro-Michael addition. In a preferred embodiment the thio-alcohol is selected from dithiothreythol, 2-hydroxy-1-ethanethiol, hydroxypropane thiol and hydroxybutane thiol.

The compound capable of participating in a Michael addition and/or retro-Michael addition may be used in a

catalytic amount, although higher amounts may be used. The molar ratio of said compound capable of participating in a Michael addition and/or retro-Michael addition to the isomers can be chosen within wide limits. Usually the ratio may be within the range of 0.00001:1 to 100:1, preferably in the range of 0.001:1 to 10:1, more preferably 0.01:9 to 0.5:1.

The ene reductase may be employed isolated from an organism or inside an organism expressing the ene reductase. In particular a micro-organism expressing the ene reductase may be used. The ene reductase may be dissolved, suspended or dispersed in a reaction medium or immobilised on a carrier. The organism expressing the may be a naturally occurring organism or a genetically modified (transgenic) organism. The organism may in particular be a micro-organism, more in particular a micro-organism selected from bacteria, fungi and yeasts.

The ene reductase usually show advantageous selectivity for an $\alpha,\beta$ unsaturation in a compound, also if one or more other C=C bonds are present in the compound.

Suitable hydrogen sources when using an ene reductase, are known in the art. Suitable sources in particular include NADPH (Nicotinamide adenine dinucleotide phosphate) and NADH (Nicotinamide adenine dinucleotide).

An ene reductase (E.C.1.3.1.x) is used. In particular, the ene reductase may be classified as groups containing enal reductases, enone reductases and enoate reductases dependent on the main substrates being converted by each of the respective ene reductase groups. In principle, any such enzyme may be used, *e.g.*, any ene reductase described in the above identified publications.

Preferred ene reductase include old yellow enzymes [EC 1.3.1.x]. Old yellow Enzymes may also be classified under E.C.1.6.99.1. However, in the framework of the invention it is in particular the ene-reductase activity that is of interest, hence, the classification of E.C 1.3.1 is given, but OYE enzymes are expressly included in the enzymes suitable for the process according to the invention. Particular preferred are such enzymes
from yeasts:

(OYE Saccharomyces carlsbergensis (GENBANK/X53597)
OYE2 Saccharomyces cerevisiae (GENBANK/L06124)
OYE3 Saccharomyces cerevisiae (GENBANK/L29279)
HYE1 Hansenula polymorpha (GENBANK/AF466188HYE1)
HYE2 Hansenula polymorpha (GENBANK/AF486188HYE2)),

from plants:

(P1 Arabidopsis thaliana (GENBANK/Z49768))

or from mammals;

(LTB4DH Rat (Genbank/NM_138863)).

The ene reductase, is preferably used in combination with a suitable cofactor regeneration system for the ene reductase which is known to those skilled in the art. Examples are the use of formate dehydrogenase combined with formate, or the use of glucose dehydrogenase combined with glucose. Catalytic amounts of cofactor generally suffice in these cofactor recycling systems.

A catalyst capable of converting both E isomer and Z isomer is a substrate unspecific catalyst, whereas catalysts capable of only converting E isomer or Z isomer are know as substrate specific. In the framework of the invention, a catalyst is in particular considered substrate specific if the conversion rate for one of the isomers (either E or Z) under process conditions is at least 10 times higher, preferably at least 100 times higher than for the other isomer. Else the catalyst is considered substrate unspecific. At least the substrate specific catalyst is usually employed under isomerising conditions, as described herein.

The ene reductase can be used isolated (solubilized, suspended or on a carrier) from an organism or in an organism, preferably a bacterium, such as *E. coli.* If desired, the organism can be genetically modified to include the ene reductase from another species. Optionally one or more other enzymes, in particular an enzyme for regenerating the reduced cofactor catalyzing the overall hydrogenation are co-expressed. In a particularly preferred method, a bacterium, such as *E. coli,* is used with co-expressed ene reductase and a co-factor regeneration enzyme for the ene reductase.

Optionally one or more other enzymes which, *e.g.*, can catalyse a further reaction of the chiral compound or catalyse isomerisation, can be co-expressed with the ene reductase.

In an advantageous method of the invention, enantioselective reduction of the carbon-carbon double bond of the $\alpha,\beta$-unsaturated compound and a further modification of the compound, takes place in a "one-pot" process. In a "one-pot" process reaction conditions are such that both reduction of the isomers and the further modification take place. In particular, in such process, an ene reductase for the reduction (plus any needed reagent, in particular a hydrogen

source, and - if desired a regeneration system) and a catalyst for such further modification (and any needed reagent, and - if desired - a regeneration system) are present simultaneously in the reaction medium also comprising the E isomer and the Z isomer of the $\alpha,\beta$-unsaturated compound.

The chiral compound produced in accordance with the invention may be a pharmaceutically active compound, an intermediate for a pharmaceutically active compound, an agrochemical or an intermediate for an agrochemical or another useful compound.

The chiral compound may be further converted into a different compound.

The chiral compound obtained by enantioselective reduction using the ene reductase comprises an aldehyde-group which is further converted into a corresponding alcohol. Reduction of the aldehyde group can be accomplished chemically or enzymatically. Enzymatic reduction can in particular be done using an alcohol dehydrogenase. Suitable reaction conditions may be based on conditions known in the art.

In an embodiment, the conversion of the carbon-carbon double bond according to the invention and the reduction of the aldehyde group are carried out in the same reaction medium.

Further conversions are also possible. In an embodiment the compound with formula (3) wherein $R_3$, $R_4$ and $R_5$ are as described above for formula (2), the hydroxyl group is subsequently substituted by a halogen atom, preferably Cl, to form a compound according to Formula (X),

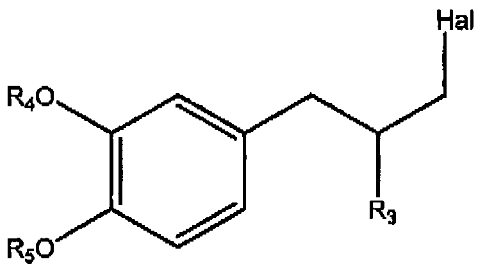

(X)

wherein $R_3$, $R_4$ and $R_5$ are as described above for formula (2), and Hal is a halogen atom.

Preferably, for compounds of formula (3) and compound of formula (X) $R_3$ is 2-propyl, $R_4$ is 3-methoxypropyl and $R_5$ is methyl,

In an especially preferred embodiment, enantioselective reduction of the carbon-carbon double bond of the $\alpha$, $\beta$-unsaturated compound and a reduction of the aldehyde group is carried out in the presence of an ene reductase, a regeneration system for the ene reductase and an enzyme capable of catalysing the reduction of the aldehyde group (which enzyme usually is different from said ene reductase). If desired, a regeneration system for the enzyme capable of catalysing the reduction of the aldehyde group may be present, which may be the same or different as the regeneration system for the ene reductase.

A compound according to Formula 3 prepared according to a process of the invention may be used for the preparation of a compound represented by Formula (XVII)

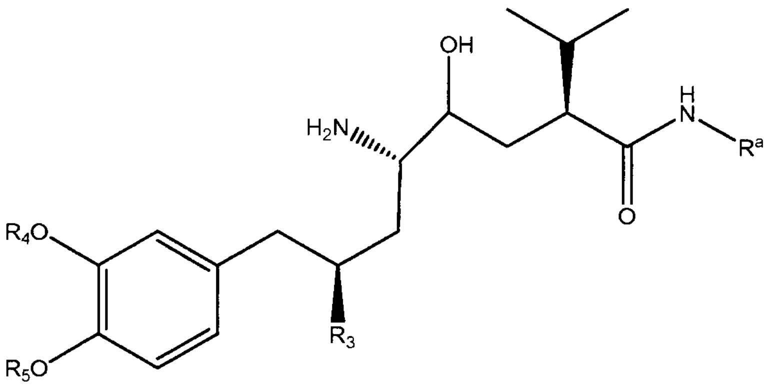

(XVII)

wherein $R_3$, $R_4$ and $R_5$ are as described above for formula (2), $R^a$ is H, or a hydrocarbons, which hydrocarbons optionally comprise one or more heteroatoms.

The invention will now be illustrated with the following examples.

Examples

Example 1:

a) Preparation of pyrrolidino-3-methylbut-1-ene (enamine)

194 g (2.25 mol) of isovaleraldehyde were diluted in 1115 ml of toluene and cooled, with stirring, to 0°C. To this solution, 190.3 g (2.68 mol) of pyrrolidine, diluted in 185.8 ml of toluene, were added dropwise, so that the reaction temperature did not rise above 5°C. Upon completion of the addition, the reaction solution was stirred for one more hour at 5°C. Thereupon the reaction mixture was heated to room temperature and the water formed was separated completely by extraction with toluene. After this, the solvent was removed through evaporation and the crude product (329.1 g; 95% of the theoretical yield) stored in a refrigerator at 4°C.

b) Reaction of enamine as prepared in Example 1a with 4-methoxy-3-(3-methoxypropoxy)-benzaldehyde (A1)

222.3 g (0.99 mol) of A1 were diluted with 240 g of 2-propanol. To this solution 321.2 g (2.31 mol) of the enamine were added, with stirring, at room temperature. The reaction mixture was then heated to 80°C and stirred at this temperature for 50 hours. To remove unreacted A1, the reaction mixture was extracted with 1170 ml of $NaHSO_3$ (40%) and 1365 ml of water for 30 minutes.

The unreacted enamine was evaporated using a rotary evaporator and entrained with 2-propanol (40 mbar, 50°C). After an aqueous extraction, 148.4 g of the product aldehyde (51.2 %) were obtained.

Example 2:

60 g (394 mmol) of isovanillin were dissolved in 200 ml of DMF and cooled to 0°C. 120 g of $Et_3N$ were added and 63 g (550 mmol) of methane sulfonyl chloride were slowly added dropwise. Thereupon the reaction mixture was extracted with EtOAc and HCl and then rotated to dryness (60°C, 10 mbar). Yield 83 g mesylated isovanillin (92% of the theoretical yield).

83 g (360 mmol) of mesylated isovanillin were dissolved in 250 ml of DMF and 250 ml of toluene and, with stirring, reacted at 60°C with 90 g (646 mmol) of enamine prepared according to Example 1a.

Thereupon the solvent was extracted using a rotary evaporator (Rotavapor).

Yield 70 g (65% of the theoretical yield).

Example 3.

Method of preparation of 2-(3-(methoxypropoxy)-4-methoxybenzyl)-3-methylbutanal by 2-electron bioreduction of 2-(3-(methoxypropoxy)-4-methoxybenzylidene)-3-methylbutanal with *E.coli* cells expressing Enone Reductase (ER), adding Glucose Dehydrogenase (GDH from *Bacillus megaterium* purchased at Jülich Chiral Solutions) for cofactor recycle, yielding highly enantiomerically enriched saturated aldehyde

The example focuses on the production of enantio-enriched saturated aldehyde under isomerising conditions starting from the E/Z mixture of 2-(3-(methoxypropoxy)-4-methoxybenzylidene)-3-methylbutanal. 1,4 dithio-DL-threitol (DTT) is used as isomerisation catalyst.

Conditions:

Atmospheric pressure, 25°C, pH=7.5 (pH adjustment with NaOH)

Ingredients needed:

2-(3-(methoxypropoxy)-4-methoxybenzylidene)-3-methylbutanal (149.4 mg oil, purity=95%, E/Z ratio= 74/26), Potassium phosphate buffer 100 mM pH=7.5 (27 ml), $NADP^+$ (25 mg),

Cell free extract (prepared via sonification) of *E.coli* TOP10 cells expressing Enone Reductase P1 from *A. thaliana* (3 ml cell free extract, equivalent with 230 mg cell wet weight, 25% over-expression of total protein), glucose dehydrogenase (400 units), glucose (200 mg), 1,4 dithio-DL-threitol (DTT, 1 ml of 1 M solution in water). All over-expression experiments were carried out following Invitrogen protocols at www.invitrogen.com for Gateway cloning.

Results:

After 24 hr 2-(3-(methoxypropoxy)-4-methoxybenzylidene)-3-methylbutanal conversion was >99%, closing the carbon balance as follows: >90% had been converted to the (R)-enantiomer of the corresponding saturated aldehyde (e.e. = 82%), <10% was converted to the corresponding saturated alcohol (due to background ADH activity of *E.coli* cells).

Example 4.

Method for the preparation of 2-(3-(methoxypropoxy)-4-methoxybenzyl)-3-methylbutanol by 4-electron bioreduction of 2-(3-(methoxypropoxy)-4-methoxybenzylidene)-3-methylbutanal with *E.coli* cells expressing Enone Reductase (ER), *E.coli* TOP10 cells expressing Alcohol Dehydrogenase (ADH), adding Glucose Dehydrogenase (GDH from *Bacillus megaterium* purchased at Jülich Chiral Solutions) for cofactor recycle, yielding highly enantiomerically enriched saturated alcohol.

The example focuses on the production of enantio-enriched saturated alcohol under isomerising conditions starting from the E/Z mixture of 2-(3-(methoxypropoxy)-4-methoxybenzylidene)-3-methylbutanal. 1,4 dithio-DL-threitol (DTT) is used as isomerisation catalyst.

Conditions:

Atmospheric pressure, 25°C, pH=7.5 (pH adjustment with NaOH)

Ingredients needed:

2-(3-(methoxypropoxy)-4-methoxybenzylidene)-3-methylbutanal (151.1 mg oil, purity=95%, E/Z ratio= 74/26), Potassium phosphate buffer 100 mM pH=7.5 (27 ml), $NADP^+$ (25 mg),

Cell free extract (prepared via sonification) of *E.coli* TOP10 cells (purchased at Invitrogen) expressing Enone Reductase P1 (3 ml cell free extract, equivalent with 230 mg cell wet weight, 25% over-expression of total protein), cell free extract (prepared via sonification) of *E.coli* TOP10 cells expressing ADH E7 (1 ml cell free extract, equivalent with 80 mg cell wet weight, 30% over-expression of total protein), glucose dehydrogenase (400 units), glucose (200 mg), 1,4 dithio-DL-threitol (DTT, 1 ml of 1 M solution in water). All over-expression experiments were carried out following Invitrogen protocols at www.invitrogen.com for Gateway cloning.

Results:

After 24 hr 2-(3-(methoxypropoxy)-4-methoxybenzylidene)-3-methylbutanal conversion was >99%, almost closing the carbon balance with the saturated alcohol (4-electron reduced product). As a result, >90% of the almost completely converted substrate had been converted to the (R)-enantiomer of the corresponding saturated alcohol (e.e. = 82%).

Example 5

The effect of an isomerising catalyst in the 2-electron bioreduction of 2-(3-(methoxypropoxy)-4-methoxybenzylidene)-3-methylbutanal with *E.coli* cells expressing Enone Reductase (ER) and added glucose Dehydrogenase (GDH from *Bacillus megaterium* purchased at Jülich Chiral Solutions) for cofactor recycle, yielding enantiomerically enriched corresponding saturated aldehyde.

The example focuses on the effect of an isomerising catalyst in the production of enantio-enriched saturated aldehyde starting from the E/Z mixture of 2-(3-(methoxypropoxy)-4-methoxybenzylidene)-3-methylbutanal.

Conditions:

Atmospheric pressure, 25°C, pH=7.5 (pH adjustment with NaOH)

Ingredients needed:

A) 2-(3-(methoxypropoxy)-4-methoxybenzylidene)-3-methylbutanal (150 mg oil, purity=95%, E/Z ratio= 74/26), potassium phosphate buffer 100 mM pH=7.5 (27 ml), $NADP^+$(25 mg),

Cell free extract (prepared via sonification) of *E.coli* TOP10 cells expressing Enone Reductase P1 (1.2 ml cell free extract, equivalent with 300 mg cell wet weight, 9% over-expression of total protein), glucose dehydrogenase (400 units), glucose (200 mg), 1,4 dithio-DL-threitol (DTT, 1 ml of 1 M solution in water, end concentration= 30mM). All over-expression experiments were carried out following Invitrogen protocols at www.invitrogen.com for Gateway cloning.

B) As A, but instead of DTT, Mercapto-ethanol is added (70 mg, end concentration= 30mM).
C) As A, but no additive.

Results:

Z-2-(3-(methoxypropoxy)-4-methoxybenzylidene)-3-methylbutanal is not converted if no isomerisation catalyst is added. The Enone Reductase does not accept the Z-isomer.

If DTT or Mercapto-ethanol is added the Z-isomer is converted via isomerisation towards to E-isomer and further by the Enone Reductase to the saturated aldehyde.

## Claims

1. Process for the preparation of a chiral compound, comprising enantioselectively reducing a carbon-carbon double bond of an $\alpha,\beta$-unsaturated compound in a mixture comprising both an E isomer and a Z isomer of the $\alpha,\beta$-unsaturated compound, wherein both E isomer and Z isomer are converted in the presence of an ene reductase, wherein the $\alpha,\beta$-unsaturated compound is a compound represented by Formula 2

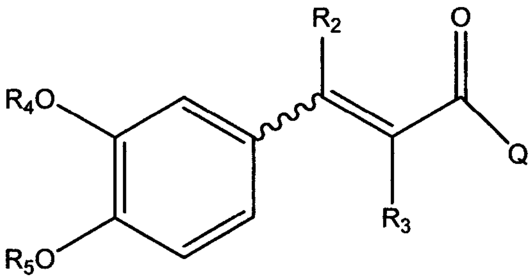

(2)

wherein $R_2$ is H; $R_3$ is an alkyl group comprising between 1 and 12 C-atoms; $R_4$ is selected from the group of hydrogen, $C_1$-$C_6$ alkyls, $C_2$-$C_6$ alkoxyalkyls and oxygen protective groups; $R_5$ is selected from the group of hydrogen, $C_1$-$C_6$ alkyl or an oxygen protective group; Q is H,
wherein the reduction is carried out under isomerising conditions, said isomerising conditions comprising the presence of an alkane thiol or an aryl thiol capable of participating in a Michael addition and a retro-Michael addition, and wherein the electron withdrawing group -(C=O)-Q is reduced,
to provide a compound with formula (3)

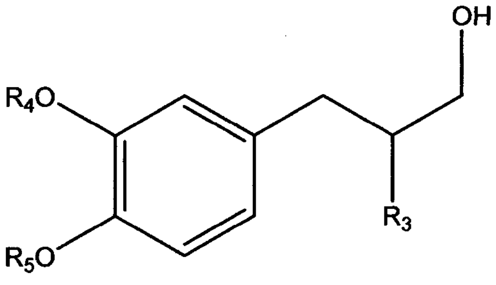

(3)

wherein $R_3$, $R_4$ and $R_5$ are as described above for formula (2), wherein the oxygen protecting group may be selected from the group of tosylate, mesylate, benzoylate, benzoate, trihydrocarbon-silyl and organic acid residues.

2. Process according to claim 1, wherein the electron withdrawing group -(C=O)-Q is reduced after said reduction of the carbon-carbon double bond.

3. Process according to claim 1, wherein the molar ratio Z isomer to E isomer in the mixture at the start of the process is in the range of 5:95 to 95:5.

4. Process according to claim 1, wherein the molar ratio Z isomer to E isomer in the mixture at the start of the process is in the range of 10:90 to 90:10.

5. Process according to claim 1, wherein the molar ratio Z isomer to E isomer in the mixture at the start of the process is in the range of 20:80 to 80:20.

6. Process according to any one of claims 1-5, wherein the chiral compound is formed with an enantiomeric excess of at least 50 %.

7. Process according to any one of claims 1-6, wherein the reduction is carried out in the presence of a cofactor regeneration system for the ene reductase.

8. Process according to any one of claims 1-7, wherein the enzyme is a substrate unspecific enzyme.

9. Process according to any one of claims 1-8, wherein the enzyme is a substrate specific enzyme.

10. Process according to any one of claims 1-9, wherein the ene reductase is selected from the group of ene reductases HYE1 (Hansenula polymorpha), HYE2 (Hansenula polymorpha), P1 (Arabidopsis thaliana) and LTB4DH (Rat).

11. Process according to any one of claims 1-10, wherein the conversion is carried out in water or an aqueous liquid.

12. Process according to claim 11, wherein the aqueous liquid comprises a cosolvent.

13. Process according to any of the claims 1-12, wherein the reduction of the carbon-carbon double bond and the reduction of the electron withdrawing group - (C=O)Q are carried out in the same reaction medium.

14. Process according to any one of claims 1-13 wherein in a compound according to formula (3) wherein $R_3$ is 2-propyl, $R_4$ is 3-methoxypropyl and $R_5$ is methyl, the hydroxyl group is subsequently substituted by a halogen atom, to form a compound according to Formula (X),

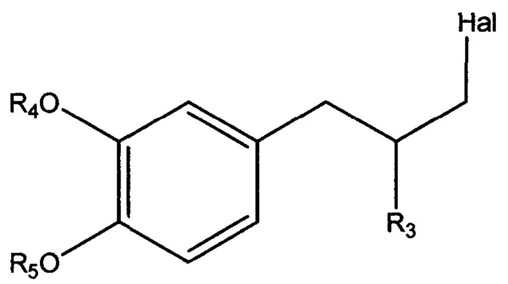

(X)

wherein $R_3$ is 2-propyl, $R_4$ is 3-methoxypropyl, $R_5$ is methyl, and Hal is a halogen atom.

15. A process according to claim 14, wherein the halogen atom is CL.

**Patentansprüche**

1. Verfahren zur Herstellung einer chiralen Verbindung, bei dem man eine Kohlenstoff-Kohlenstoff-Doppelbindung einer α,β-ungesättigten Verbindung in einer Mischung, die sowohl ein E-Isomer als auch ein Z-Isomer der α,β-ungesättigten Verbindung umfasst, enantioselektiv reduziert, wobei sowohl das E-Isomer als auch das Z-Isomer in Gegenwart einer Enreduktase umgewandelt werden, wobei es sich bei der α,β-ungesättigten Verbindung um eine Verbindung gemäß Formel 2 handelt,

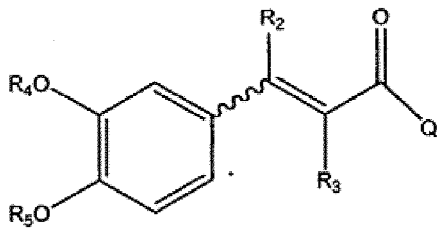

(2)

worin $R_2$ H bedeutet; $R_3$ eine Alkylgruppe mit zwischen 1 und 12 Kohlenstoffatomen bedeutet; $R_4$ aus der Gruppe Wasserstoff, $C_1$-$C_6$-Alkyle, $C_2$-$C_6$-Alkoxyalkyle und Sauerstoffschutzgruppen ausgewählt ist; $R_5$ aus der Gruppe Wasserstoff, $C_1$-$C_6$-Alkyl oder Sauerstoffschutzgruppe ausgewählt ist; Q H bedeutet;
wobei die Reduktion unter Isomerisierungsbedingungen durchgeführt wird, wobei die Isomerisierungsbedingungen das Vorliegen eines Alkanthiols oder eines Arylthiols, das fähig ist, an einer Michael-Addition und einer retro-Michael-Addition teilzunehmen, umfassen und wobei die elektronenziehende Gruppe -(C=0)-Q reduziert wird, wodurch man zu einer Verbindung der Formel (3) gelangt,

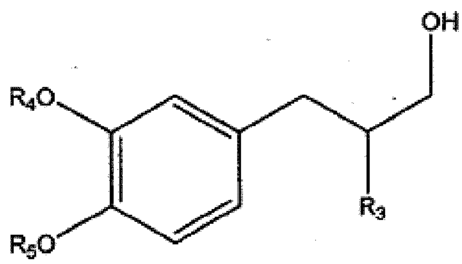

(3)

worin $R_3$, $R_4$ und $R_5$ wie oben für Formel (2) beschrieben sind, wobei die Sauerstoffschutzgruppe aus der Gruppe Tosylat, Mesylat, Benzoylat, Benzoat, Trikohlenwasserstoffsilyl und organischen Resten ausgewählt sein kann.

**2.** Verfahren nach Anspruch 1, wobei die elektronenziehende Gruppe - (C=O) -Q nach der Reduktion der Kohlenstoff-Kohlenstoff-Doppelbindung reduziert wird.

**3.** Verfahren nach Anspruch 1, wobei das Molverhältnis von Z-Isomer zu E-Isomer in der Mischung am Beginn des Verfahrens im Bereich 5:95 bis 95:5 liegt.

**4.** Verfahren nach Anspruch 1, wobei das Molverhältnis von Z-Isomer zu E-Isomer in der Mischung am Beginn des Verfahrens im Bereich 10:90 bis 90:10 liegt.

**5.** Verfahren nach Anspruch 1, wobei das Molverhältnis von Z-Isomer zu E-Isomer in der Mischung am Beginn des Verfahrens im Bereich 20:80 bis 80:20 liegt.

**6.** Verfahren nach einem der Ansprüche 1-5, wobei die chirale Verbindung mit einem Enantiomerenüberschuss von mindestens 50% gebildet wird.

**7.** Verfahren nach einem der Ansprüche 1-6, wobei die Reduktion in Gegenwart eines Kofaktorregenerationssystems für die Enreduktase erfolgt.

**8.** Verfahren nach einem der Ansprüche 1-7, wobei es sich bei dem Enzym um ein substratunspezifisches Enzym handelt.

**9.** Verfahren nach einem der Ansprüche 1-8, wobei es sich bei dem Enzym um ein substratspezifisches Enzym handelt.

**10.** Verfahren nach einem der Ansprüche 1-9, wobei die Enreduktase aus der Gruppe HYE1-Enreduktase (Hansenula polymorpha), HYE2-Enreduktase (Hansenula polymorpha), P1-Enreduktase (Arabidopsis thaliana) und LTB4DH-Enreduktase (Ratte) ausgewählt ist.

11. Verfahren nach einem der Ansprüche 1-10, wobei die Umwandlung in Wasser oder in einer wässrigen Flüssigkeit erfolgt.

12. Verfahren nach Anspruch 11, wobei die wässrige Flüssigkeit ein Hilfslösungsmittel umfasst.

13. Verfahren nach einem der Ansprüche 1-12, wobei die Reduktion der Kohlenstoff-Kohlenstoff-Doppelbindung und die Reduktion der elektronenziehenden Gruppe - (C=O) Q in demselben Reaktionsmedium erfolgen.

14. Verfahren nach einem der Ansprüche 1-13, wobei in einer Verbindung der Formel (3), worin $R_3$ 2-Propyl bedeutet, $R_4$ 3-Methoxypropyl bedeutet und $R_5$ Methyl bedeutet, die Hydroxylgruppe anschließend durch ein Halogenatom ersetzt wird, wodurch man zu einer Verbindung der Formel (X) gelangt,

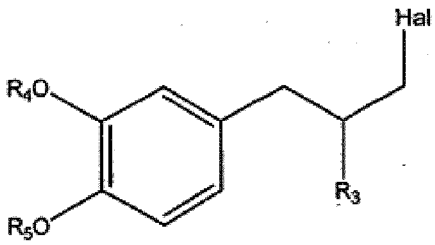

(X)

worin $R_3$ 2-Propyl bedeutet, $R_4$ 3-Methoxypropyl bedeutet, $R_5$ Methyl bedeutet und Hal ein Halogenatom bedeutet.

15. Verfahren nach Anspruch 14, wobei es sich bei dem Halogenatom um Cl handelt.

## Revendications

1. Procédé de préparation d'un composé chiral, comprenant la réduction énantiosélective d'une double liaison carbone-carbone d'un composé α,β-insaturé dans un mélange comprenant tant un isomère E qu'un isomère Z du composé α,β-insaturé, dans lequel les deux isomères E et Z sont convertis en présence d'une ène-réductase, dans lequel le composé α,β-insaturé est un composé représenté par la Formule 2

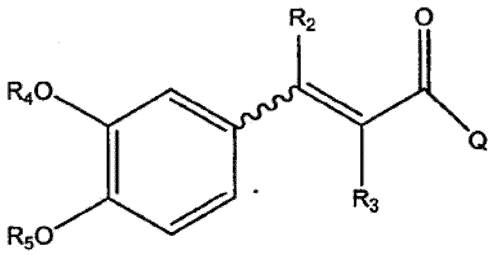

(2)

dans laquelle $R_2$ est H ; $R_3$ est un groupe alkyle ayant de 1 à 12 atomes de carbone ; $R_4$ est choisi dans le groupe consistant en l'atome d'hydrogène, les groupes alkyle en $C_1$-$C_6$, alcoxyalkyle en $C_2$-$C_6$ et les groupes protecteurs vis-à-vis de l'oxygène ; $R_5$ est choisi dans le groupe consistant en l'atome d'hydrogène, les groupes alkyle en $C_1$-$C_6$ et les groupes protecteurs vis-à-vis de l'oxygène ; Q est H,
dans lequel la réduction est mise en oeuvre dans des conditions d'isomérisation, lesdites conditions d'isomérisation comprenant la présence d'un alcanethiol ou d'un arylthiol capable de participer à une addition de Michael et à une addition de rétro-Michael, et dans lequel le groupe attracteur d'électrons -(C=O)-Q est réduit,
pour donner un composé de formule (3)

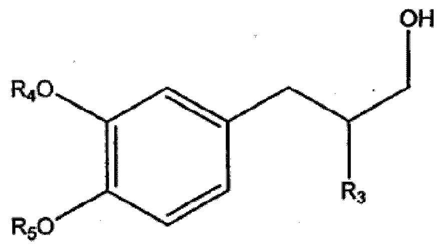

(3)

dans laquelle $R_3$, $R_4$ et $R_5$ sont tels que décrits ci-dessus pour la formule (2), le groupe protecteur vis-à-vis de l'oxygène pouvant être choisi dans le groupe consistant en les résidus tosylate, mésylate, benzoylate, benzoate, trihydrocarbosilyle et acide organique.

2. Procédé selon la revendication 1, dans lequel le groupe attracteur d'électrons -(C=O)-Q est réduit après ladite réduction de la double liaison carbone-carbone.

3. Procédé selon la revendication 1, dans lequel le rapport en moles de l'isomère Z à l'isomère E dans le mélange au début du procédé est compris dans la plage de 5:95 à 95:5.

4. Procédé selon la revendication 1, dans lequel le rapport en moles de l'isomère Z à l'isomère E dans le mélange au début du procédé est compris dans la plage de 10:90 à 90:10.

5. Procédé selon la revendication 1, dans lequel le rapport en moles de l'isomère Z à l'isomère E dans le mélange au début du procédé est compris dans la plage de 20:80 à 80:20.

6. Procédé selon l'une quelconque des revendications 1 à 5, dans lequel le composé chiral est formé avec un excès énantiomérique d'au moins 50 %.

7. Procédé selon l'une quelconque des revendications 1 à 6, dans lequel la réduction est mise en oeuvre en présence d'un système de régénération de cofacteur pour l'ène-réductase.

8. Procédé selon l'une quelconque des revendications 1 à 7, dans lequel l'enzyme est une enzyme non spécifique de substrat.

9. Procédé selon l'une quelconque des revendications 1 à 8, dans lequel l'enzyme est une enzyme spécifique de substrat.

10. Procédé selon l'une quelconque des revendications 1 à 9, dans lequel l'ène-réductase est choisie dans le groupe consistant en les ène-réductases HYE1 (Hansenula polymorpha), HYE2 (Hansenula polymorpha), P1 (Arabidopsis thaliana) et LTB4DH (Rat).

11. Procédé selon l'une quelconque des revendications 1 à 10, dans lequel la conversion est mise en oeuvre dans de l'eau ou dans un liquide aqueux.

12. Procédé selon la revendication 11, dans lequel le liquide aqueux comprend un co-solvant.

13. Procédé selon l'une quelconque des revendications 1 à 12, dans lequel la réduction de la double liaison carbone-carbone et la réduction du groupe attracteur d'électrons -(C=O)Q est mise en oeuvre dans le même milieu réactionnel.

14. Procédé selon l'une quelconque des revendications 1 à 13, dans lequel, dans un composé selon la formule (3) dans laquelle $R_3$ est le groupe 2-propyle, $R_4$ est le groupe 3-méthoxypropyle et $R_5$ est le groupe méthyle, le groupe hydroxyle est ultérieurement substitué par un atome d'halogène, pour former un composé de formule (X)

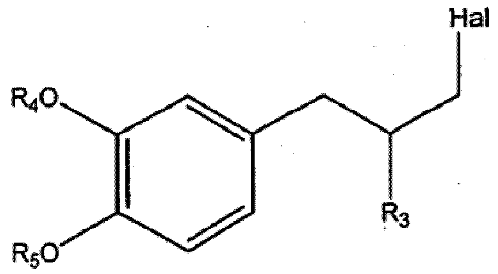

(X)

dans laquelle $R_3$ est le groupe 2-propyle, $R_4$ est le groupe 3-méthoxypropyle, $R_5$ est le groupe méthyle et Hal est un atome d'halogène.

**15.** Procédé selon la revendication 14, dans lequel l'atome d'halogène est Cl.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FARDELONE et al.** *J. Mol. Catal. B: Enzymatic,* 2004, vol. 29, 41-45
- **FERRABOSCHI et al.** *Tetrahedron:Asymmetry,* 1999, vol. 10, 2639
- **MANO et al.** *Plant & Cell Physiology,* 2002, vol. 43 (12), 1445-1455
- **HALL et al.** *Angewandte Chemie,* 2007, vol. 46, 3934-3937
- **MÜLLER et al.** *Biotechnology and Bioengineering,* 2007, vol. 98 (1), 22-29